# EUROPEAN PATENT SPECIFICATION

(11) **EP 4 003 451 B1**
(45) Date of publication and mention of the grant of the patent: **18.12.2024**
(21) Application number: 20743701.3
(22) Date of filing: 24.07.2020
(51) Int. Cl.: A61M 1/34, A61K 47/00, A61M 1/36

(54) **DEVICE FOR EXTRACORPOREAL LOWERING OF ASYMMETRIC DIMETHYLARGININE AND MONOMETHYL ARGININE LEVELS IN HUMAN BLOOD**
VORRICHTUNG ZUR EXTRAKORPORALEN SENKUNG VON ASYMMETRISCHEN DIMETHYLARGININ- UND ARGININSPIEGELN IN MENSCHLICHEM BLUT
DISPOSITIF D'ABAISSEMENT EXTRACORPOREL DES NIVEAUX DE DIMÉTHYLARGININE ASYMÉTRIQUE ET DE MONOMÉTHYLE ARGININE DANS LE SANG HUMAIN

(30) Priority: 26.07.2019 EP 19188652
(43) Date of publication of application: 01.06.2022
(73) Proprietor: Technische Universität Dresden, 01069 Dresden (DE)
(72) Inventor: RODIONOV, Roman N., 01309 Dresden (DE); TSELMIN, Sergey, 01309 Dresden (DE); BORNSTEIN, Stefan R., 01309 Dresden (DE)
(74) Representative: Hertin und Partner Rechts- und Patentanwälte PartG mbB
(86) International application number: PCT/EP2020/070919
(87) International publication number: WO 2021/018758

(56) References cited:
- US-A1- 2016 145 615
- R. N. RODIONOV ET AL: "Human Alanine-Glyoxylate Aminotransferase 2 Lowers Asymmetric Dimethylarginine and Protects from Inhibition of Nitric Oxide Production", JOURNAL OF BIOLOGICAL CHEMISTRY, vol. 285, no. 8, 19 February 2010 (2010-02-19), pages 5385 - 5391, XP055006527, ISSN: 0021-9258, DOI: 10.1074/jbc.M109.091280
- CZARNECKA ANNA ET AL: "Asymmetric Dimethylarginine and Hepatic Encephalopathy: Cause, Effect or Association?", NEUROCHEMICAL RESEARCH, PLENUM PRESS, NEW YORK, US, vol. 42, no. 3, 25 November 2016 (2016-11-25), pages 750 - 761, XP036191935, ISSN: 0364-3190, [retrieved on 20161125], DOI: 10.1007/S11064-016-2111-X
- DENNIS J. STUEHR: "Enzymes of the L-arginine to nitric oxide pathway", THE JOURNAL OF NUTRITION, vol. 134, no. 10, 1 October 2014 (2014-10-01), US, pages 2748 - 2751, XP055438922, ISSN: 0022-3166, DOI: 10.1007/s00726-014-1825-9

## Description

The invention relates to the field of extracorporeal blood treatment, in particular selective lowering levels of asymmetric dimethylarginine (ADMA) and/or monomethyl arginine (MMA) in the blood or blood plasma of a patient, and related devices and methods.

The invention relates to a blood treatment device configured to lower the amount of asymmetric dimethylarginine (ADMA) and/or monomethyl arginine (MMA) in the blood or blood plasma of a person in need thereof in an extracorporeal blood circuit, wherein the device comprises a matrix, and wherein said matrix comprises an enzyme configured to metabolize ADMA and/or MMA. The invention further relates to an extracorporeal blood circuit comprising a blood treatment device of the invention and to the blood treatment device for use as a medicament in the treatment and/or prevention of a medical condition associated with elevated asymmetric dimethylarginine (ADMA) and/or monomethyl arginine (MMA) levels and/or a medical condition associated with impaired nitric oxide (NO) production and/or bioavailability.

### BACKGROUND OF THE INVENTION

ADMA is an endogenous dimethylated derivative of L-arginine, which can inhibit nitric oxide (NO) production in vitro and in vivo. Systemic levels of ADMA are elevated in major cardiovascular, metabolic and renal pathologies associated with impaired NO bioavailability, such as hypertension, congestive heart failure, atherosclerosis, stroke, hypercholesterolemia, hyperhomocysteinemia, peripheral arterial disease, preeclampsia, chronic kidney disease, and diabetes mellitus [1]. Elevation of plasma ADMA levels in animal models leads to impairment of NO production resulting in endothelial dysfunction, increased systemic vascular resistance, and elevated blood pressure [2] suggesting that ADMA may mediate cardiovascular damage in the diseases associated with increased ADMA levels.

The major pathway for catabolism of ADMA is hydrolysis to citrulline and methylamines catalyzed by the enzyme dimethylarginine dimethylaminohydrolase (DDAH). There are two isoforms of DDAH (DDAH1 and DDAH2) in mammals, each encoded by a separate gene. DDAH1 is thought to be the main ADMA-metabolizing isoform of DDAH [3]. Upregulation of DDAH1 was shown to be protective in multiple experimental models of cardiovascular pathologies. Transgenic DDAH1 overexpression in mice lowers systemic ADMA levels, increases basic and stimulated NO production [4], improves angiogenesis in ischemic hind limb model [5], increases insulin sensitivity [6] and protects from myocardial and renal ischemia-reperfusion injury [7,8]. DDAH1 transgenic (DDAH1 Tg) mice are also protected from atherosclerosis [9], hyperhomocysteinemia-induced vascular injury [10], renal injury in a murine model of hypertension [11] and angiotensin-II-induced myocardial and aortic remodeling.

Diverse protective effects of DDAH1 in animal models indicate that subsequent lowering of ADMA level in human plasma through upregulation of DDAH1 activity might be a promising therapeutic strategy in cardiovascular, metabolic and renal diseases, amongst others.

Multiple drug screens for DDAH1 activators have been unsuccessful, potentially, because DDAH1 is a small protein, which might have no sites for allosteric regulation. At present, there are no available selective ADMA-lowering therapies available in the treatment of disease.

Even though currently available extracorporeal methods can non-selectively reduce ADMA and MMA in blood, they also lower L-Arginine, which counterbalances the positive effects of ADMA- and MMA-lowering. Furthermore, nonselective lowering of ADMA and MMA does not allow establishing the causative role of these endogenous compounds in development of cardiovascular, metabolic and renal damage in humans.

The lack of selective ADMA- and MMA-lowering therapies for restoration of NO bioavailability in cardiovascular, metabolic and renal pathologies is the unmet clinical need, which will be addressed by our invention.

### SUMMARY OF THE INVENTION

In light of the prior art, the technical problem underlying the present invention is to provide alternative and/or improved and/or selective means for treating medical conditions associated with elevated asymmetric dimethylarginine (ADMA) and/or monomethyl arginine (MMA) levels, or impaired nitric oxide (NO) production and/or bioavailability. Another object of the invention is the provision of alternative, or improved, or more selective means for lowering the amounts of ADMA and/or MMA in a subject. The present invention seeks to provide such means, while avoiding the disadvantages known in the prior art.

The problem is solved by the features of the independent claim which delimit the scope of the present invention. Preferred embodiments of the present invention are provided by the dependent claims.

The invention therefore relates to an enzyme that metabolizes asymmetric dimethylarginine (ADMA) and/or monomethyl arginine (MMA), selected from dimethylarginine dimethylaminohydrolase (DDAH) and/or alanine-glyoxylate aminotransferase 2 (AGXT2), for use as a medicament in the treatment and/or prevention of a medical condition associated with elevated asymmetric dimethylarginine (ADMA) and/or monomethyl arginine (MMA) levels in the blood or blood plasma of a subject, compared to healthy controls, and/or for use in the treatment of a medical condition associated with impaired nitric oxide (NO) production and/or bioavailability and/or a medical condition, in which an increase of NO production and/or bioavailability is expected to be protective.

In one aspect, the invention therefore relates to a blood treatment device configured to lower the amount of asymmetric dimethylarginine (ADMA) and/or monomethyl arginine (MMA) in the blood or blood plasma of a person in need thereof in an extracorporeal blood circuit, wherein the device comprises a matrix, and wherein said matrix comprises an enzyme configured to metabolize ADMA and/or MMA.

Methylated derivatives of arginine asymmetric dimethylarginine (ADMA) and monomethyl arginine (MMA) are endogenously produced inhibitors of nitric oxide synthases (NOS). Nitric oxide (NO) is the most potent vasodilator that is synthesized from the amino acid L-arginine. Asymmetric dimethylarginine (ADMA) and monomethyl arginine (MMA) are natural competitive inhibitors of the endogenous nitric oxide synthase (NOS). ADMA is more prevalent than MMA and therefore has been preferentially assessed in clinical studies. An increase in ADMA levels is often observed in subjects with classical or novel cardiovascular risk factors such as hypercholesterolemia, insulin resistance, diabetes mellitus, hypertension and chronic kidney disease. ADMA has also shown to be an excellent predictor of mortality in selected patient populations as well as in the general population.

At present, to the knowledge of the inventors, there are no medical techniques or selective drugs available for selective lowering of ADMA or MMA levels in human blood plasma. The present invention is therefore particularly useful in achieving a reduction of primary cardiovascular risk, as well as more effective treatment of patients with already developed acute and chronic cardiovascular disorders, for example myocardial infarction in its acute phase, e.g. in an intensive care unit.

The extracorporeal lowering of human blood plasma ADMA and MMA levels by means of a selective column containing an appropriate enzyme, such as preferably DDAH (DDAH1 or DDAH2) and/or AGXT2, enables the reduction of primary and secondary cardiovascular, metabolic and renal risk as well as providing an additional therapeutic option for acute and chronic cardiovascular, metabolic and renal diseases.

To the knowledge of the inventors, the present invention represents the first approach for selective lowering of ADMA or MMA levels in human blood or plasma. The present approach therefore represents a novel and previously unsuggested apheresis technique based not on the classic mechanisms of adsorption, filtration or precipitation, but based on the chemical elimination/metabolism of a target substrate by an enzyme. This approach may therefore be termed enzymatic apheresis, or a blood treatment device based on enzymatic treatment of blood or plasma.

The present invention is associated with surprising benefits associated with the immobilized extracorporeal approach. The invention is based on isolating and immobilizing the desired enzymatic activity in a blood treatment device, showing a significant departure from traditional apheresis approaches. A skilled person could not have assumed that immobilization of the enzymes as described herein would lead to significant reduction of the ADMA or MMA levels in human blood or plasma.

The present invention is of benefit, as even if DDAH (DDAH1 or DDAH2)/AGXT2 activating drugs ever are developed, the present extracorporeal approach is advantageous due to avoidance of potential systemic side effects of endogenous DDAH (DDAH1 or DDAH2)/AGXT2 activation. The extracorporeal approach isolates and restricts the active medical effect outside the body in a localized and effective manner, thereby avoiding off-target side effects, or side effects caused for example due to the unwanted stimulation of the enzymatic activity in organs or tissues in which the activity is unwanted or unnecessary.

In one embodiment, the invention relates to a blood treatment device as described herein, wherein the enzyme configured to metabolize asymmetric dimethylarginine (ADMA) and/or monomethyl arginine (MMA) is dimethylarginine dimethylaminohydrolase (DDAH).

Dimethylarginine dimethylaminohydrolase is an enzyme, which has two isoforms, namely 1 and 2 (DDAH1 or 2), that are encoded by 2 different genes. DDAH1 was initially identified as the enzyme degrading ADMA and MMA. Studies have demonstrated that loss-of function DDAH1 mutations are associated with increases in the occurrence of coronary heart disease, thrombosis, and stroke [13]. DDAH2 was also reported to have enzyme activity for degrading ADMA and MMA in vitro that was similar to that of DDAH1 [14].

In one embodiment, the dimethylarginine dimethylaminohydrolase (DDAH) comprises or consists of isoform 1 (DDAH1) and/or isoform 2 (DDAH2).

In preferred embodiments, DDAH1 is employed, as it has been shown that DDAH1 is the critical enzyme for degrading the cardiovascular risk factor ADMA in vivo [3]. DDAH2 may however be employed alternatively or in combination, as the required in vitro activity has been detected for isoform 2.

In one embodiment, the dimethylarginine dimethylaminohydrolase (DDAH) is immobilized to the matrix and configured to hydrolyze asymmetric dimethylarginine (ADMA) and/or monomethyl arginine (MMA) by catabolism of ADMA and/or MMA to L-citrulline in blood or blood plasma.

The metabolism of ADMA occurs via hydrolytic degradation to citrulline and dimethylamine by DDAH. The enzyme is therefore immobilized using any means suitable to maintain this activity in the extracorporeal approach described herein. Further specific, non-limiting, examples of immobilization techniques are provided below.

In one embodiment, the invention relates to a blood treatment device as described herein, wherein the enzyme configured to metabolize asymmetric dimethylarginine (ADMA) and/or monomethyl arginine (MMA) is alanine-glyoxylate aminotransferase 2 (AGXT2).

AGXT2 has been shown to metabolize asymmetric dimethylarginine (ADMA) via transamination to alpha-keto-delta-(NN-dimethylguanidino) valeric acid (ADGV, also abbreviated as DMGV). The involvement of AGXT2 in regulation of NO production, including its role in regulating blood pressure, has been shown previously [15], [16].

In one embodiment, the alanine-glyoxylate aminotransferase 2 (AGXT2) is immobilized to the matrix and configured to metabolize asymmetric dimethylarginine (ADMA) and/or monomethyl arginine (MMA) via transamination to alpha-keto-delta-(NN-dimethylguanidino) valeric acid (ADGV) and alpha-keto-delta-(N-monomethylguanidino) valeric acid (MGV), respectively, in blood or blood plasma.

The enzyme is therefore immobilized using any means suitable to maintain this activity in the extracorporeal approach described herein. Further specific, non-limiting, examples of immobilization techniques are provided below.

In some embodiments, a preferred sequence of DDAH1 is according to SEQ ID NO 1.

In some embodiments, a preferred sequence of DDAH2 is according to SEQ ID NO 2.

In some embodiments, a preferred sequence of AGXT2 is according to SEQ ID NO 3.

Variation in length of the amino acid sequences as described herein is also encompassed by the present invention. A skilled person is capable of providing amino acid sequence variants that are longer or shorter than SEQ ID NO 1-3, which will still exhibit sufficient similarity to the specific proteins described herein in order to provide the outcomes desired. For example, shorter variants of SEQ ID NO 1-3 comprising 10, 20, 30, 40, or up to 50 amino acids less than the full-length form may also enable effective ADMA and/or MMA metabolism, as described herein. Fragments of SEQ ID NO 1-3 are therefore also considered. Additionally, longer variants of SEQ ID NO 1-3 comprising 10, 20, 30, 40, or up to 50 amino acids of any given additional sequence more than the natural sequences of SEQ ID NO 1-3 may also enable effective outcomes, as described herein.

In other embodiments of the invention, the DDAH or AGXT2 protein employed may comprise or consist of an amino acid sequence with at least 50%, 60%, 70%, 80%, 90% or 95% sequence identity to SEQ ID NO 1-3. Preferably the sequence variant comprises at least 80%, 90%, 91, 92, 93, 94, 95, 96, 97, 98 or 99% sequence identity to SEQ ID NO 1-3 and preferably exhibits functional analogy to the specific human enzymes described herein. Functional analogy is assessed via determining the same or a similar enzymatic effect of metabolizing ADMA and/or MMA as described herein. Suitable in vitro assays for determining the desired metabolism achieved by any given enzyme, preferably DDAH1, DDAH2, or AGXT2, are known to a skilled person.

In one embodiment, the invention relates to a blood treatment device as described herein, wherein the matrix comprises a support to which dimethylarginine dimethylaminohydrolase (DDAH) and/or alanine-glyoxylate aminotransferase 2 (AGXT2) is bound, wherein the support comprises or consists of a material selected from the group consisting of a resin, bead, fiber mat, non-woven material, porous foam and membrane, such as a tubular, hollow-fiber or flat sheet membrane.

Various device formats and materials are known to a skilled person in the field of extracorporeal blood treatment and may be applied in the context of the present invention. Possible device formats relate for example, without limitation, to membrane dialyzers and/or "adsorption" cartridges. The "adsorption" cartridge, employed in traditional extracorporeal approaches to bind and remove unwanted blood components, typically comprises a matrix selected from a resin or non-woven material, either of which is functionalized with a ligand, and in the case of the present invention, with an enzyme. The materials used in the support of such membranes and/or cartridges are known to a skilled person and can be selected accordingly. Further details are provided below.

In one embodiment, the support and/or matrix comprises or consists of at least one polymer, preferably selected from the group consisting of alginate, chitosan, chitin, collagen, carrageenan, gelatin, starch, pectin, cellulose, agarose, dextrose and sepharose; an inorganic material, preferably selected from the group consisting of ceramics, celite, silica, glass, activated carbon and charcoal; or a synthetic polymer, as described in more detail below.

In one embodiment, the blood treatment device as described herein is characterized in that the support is a hollow fiber membrane, fiber mat or flat sheet membrane, which is composed of at least one polysaccharide derivative or synthetic polymer, examples of which are provided below.

In one embodiment, the blood treatment device as described herein is characterized in that the support is a non-woven material composed of at least one biopolymer selected from polysaccharide, polylactic acid (PLA) or polycaprolactone (PCL), or of at least one inorganic material selected from the group consisting of TiO₂, SiO₂ or Al₂O₃, or from at least one synthetic polymer, examples of which are provided below.

In one embodiment, the blood treatment device as described herein is characterized in that the blood treatment device is a column, containing an immobilized enzyme or an adsorption column, e.g. a non-membrane support, preferably present as a resin, as beads or as a non-woven material, which is or is configured for being perfused with whole blood or separated blood plasma. In some embodiments, the blood treatment device is essentially in the form of, of dimensions and/or materials, known in the field as an adsorption cartridge, but without an adsorption function. Rather, columns typically used as adsorption cartridges (for example to bind unwanted antibodies or other proteins from blood) can be employed here not to adsorb but to enzymatically convert asymmetric dimethylarginine (ADMA) and/or monomethyl arginine (MMA).

The device can therefore be or comprise a column comprising a matrix selected from a suitable material or those materials disclosed herein, either of which is functionalized with an enzyme configured for metabolizing ADMA or MMA, such as those described herein. Such a device can be a member of an extracorporeal circuit for extracorporeal blood treatment, configured to provide hemodialysis or plasmapheresis. The device can be the sole blood treatment device within the blood circuit or can be located, for example, upstream or downstream of a dialyzer in a hemodialysis (hemofiltration) circuit or can alternatively be immediately connected to a dialyzer, wherein the device is configured to be perfused with whole blood. The device can also be a member of an extracorporeal plasmapheresis circuit, wherein the device is perfused with blood plasma.

In one embodiment, the dimethylarginine dimethylaminohydrolase (DDAH) and/or alanine-glyoxylate aminotransferase 2 (AGXT2) is covalently bound to the matrix, such as via cross-linking.

Covalent binding is preferred to avoid the risk of leaching of the enzyme from the matrix into the blood or blood plasma of the patient. Methods for covalent binding of an enzyme to the matrix are provided below and are known to a skilled person. In some embodiments, the enzyme is covalently bound to the matrix by treatment of the matrix and/or enzyme using for example a carbodiimide compound, such as 1-Ethyl-3-(3-dimethylaminopropyl) carbodiimide (EDC). Further examples of immobilization chemistries are presented below.

In other embodiments, the dimethylarginine dimethylaminohydrolase (DDAH) and/or alanine-glyoxylate aminotransferase 2 (AGXT2) is noncovalently bound to the matrix, such as via ionic interactions or via an affinity tag.

The selection of suitable materials and methods for noncovalent attachment are known to a skilled person. The ionic interaction between matrix and/or support and the enzyme is preferably configured such that ADMA and MMA are effectively metabolized, and the enzyme is maintained within the blood treatment device, thereby avoiding free enzyme entering the patient's blood or plasma. Affinity tags may also be employed.

In one embodiment the invention relates to a blood treatment device as described herein, wherein the blood treatment device is located in an extracorporeal blood circuit through which the blood of the patient passes, wherein said circuit comprises means for transporting blood from the patient's vascular system to the blood treatment device at a defined flow rate and for returning the treated blood back to the patient.

In one embodiment, the extracorporeal blood circuit in which the blood treatment device is located further comprises a plasma separator, such as a plasma filter or centrifuge-based plasma separation system or combination thereof (for example a disk separator), which allows for the separation of plasma from the blood, and wherein the blood treatment device is located downstream of a plasma outlet of the plasma separator.

This embodiment is designed to effectively reduce the levels of ADMA and MMA in the patient's plasma after having been separated from whole blood. In some embodiments, the plasma is either rejoined with the remaining components of the whole blood or re-infused into the subject after enzymatic treatment by the immobilized enzyme in the blood treatment device.

In one embodiment, the extracorporeal blood circuit in which the blood treatment device is located further comprises a hemofilter, for the dialysis of blood, which is located upstream or downstream of the blood treatment device, or wherein the blood treatment device is a hemofilter for the dialysis of blood.

In one embodiment, the device can be designed as a lumen or hollow fiber membrane filter or dialyzer wherein the membrane constitutes the support to which the enzyme is bound. The membrane can be a hemodialysis membrane as commonly known in the art that is additionally functionalized with an enzyme to reduce levels of ADMA and/or MMA, either on its inner lumen side or alternatively on the outer side of the fibers. In one embodiment, the device can be a hemodialysis filter that comprises a resin, e.g. in sponge form, which is functionalized with the enzyme. In other embodiments, the device is a plasma separating filter, configured to separate plasma from whole blood, whereby the enzyme is immobilized on the filter on the side at which plasma is released from the filter, thereby immediately treating the plasma in the desired fashion. In a further aspect, the invention relates to an extracorporeal blood circuit comprising a blood treatment device as described herein, wherein the extracorporeal blood circuit comprises means for transporting blood or blood plasma from the patient's vascular system to the blood treatment device at a defined flow rate and means for returning the treated blood or blood plasma back to the patient.

In some embodiments, the extracorporeal blood circuit comprises additional components such as an intravascular entry point, tubes, filters, a pump and monitors, as is commonly present in such a circuit, and which are well-known to a skilled person.

In a further aspect, the invention relates to the blood treatment device as described herein for use as a medicament in the treatment and/or prevention of:
a. a medical condition associated with elevated asymmetric dimethylarginine (ADMA) and/or monomethyl arginine (MMA) levels in the blood or blood plasma of a subject, compared to healthy controls, and/or
b. a medical condition associated with impaired nitric oxide (NO) production and/or bioavailability, compared to healthy controls.

According to one aspect of the disclosure which is not part of the invention the present disclosure which is not part of the invention provides a method of treating or ameliorating at least one symptom of such a disorder. In some embodiments, the method comprises reducing the risk of the subject worsening in condition and/or of developing a medical condition as disclosed herein.

It is therefore a further object of the present disclosure which is not part of the invention to provide a method of treating, ameliorating, reducing the risk of developing and/or reducing the risk of developing worsening symptoms of such a disorder in a patient, wherein the method comprises the step of extracorporeally lowering the level of ADMA and/or MMA from the patient by passing the blood or the blood plasma of the patient over a matrix which is configured to metabolize ADMA and/or MMA, thereby lowering their levels or removing them from the blood of the patient.

In further disclosure which is not part of the invention of the medical use or method of treatment, the medical condition is:
a. a vascular and/or cardiac disease, preferably selected from the group consisting of coronary arterial disease, peripheral arterial disease, cerebral arterial disease, heart failure, dyslipidemias, arterial hypertension, pulmonary hypertension, eclampsia, digital ulcera, erectile dysfunction, migraines, traumatic vascular injuries, transplant vasculopathies, microvascular injuries, myocardial remodeling and vascular remodeling, ischemia-reperfusion injuries and/or vasospastic diseases,
b. vascular and cardiac complications of metabolic diseases, such as diabetes mellitus, dyslipidemias, hyperhomocysteinemia and/or hyperuricemia, and/or
c. a hepatic and/or renal disease.

With respect to the above and the detailed description of the invention, all features disclosed with respect to the blood treatment device, the extracorporeal blood circuit, the medical use are considered disclosed in the context of each aspect, such that the features describing any one aspect may be considered to describe other aspects of the invention.

### DETAILED DESCRIPTION OF THE INVENTION

The present invention is based on the insight that asymmetric dimethylarginine (ADMA) and/or monomethyl arginine (MMA) can be metabolized in the blood or blood plasma of a patient in need thereof by an extracorporeal approach employing a blood treatment device in which an enzyme suitable for metabolizing ADMA and MMA is immobilized.

All terms are to be given their ordinary technical meaning, unless otherwise described herein.

### Asymmetric dimethylarginine (ADMA), Monomethyl arginine (MMA) and Nitric oxide (NO):

Asymmetric dimethylarginine (ADMA) is a naturally occurring chemical found in blood plasma. It is a metabolic by-product of protein modification processes in the cytoplasm of human cells.

ADMA interferes with L-arginine in the production of nitric oxide (NO), a key chemical involved in normal endothelial function and cardiovascular health.

Monomethyl arginine (MMA), also known as N-Methylarginine or N-monomethyl-L-arginine, is an inhibitor of nitric oxide synthase. It is a methyl derivative of the amino acid arginine and is known to have an inhibiting effect on vasodilation.

Dimethylarginines are the result of the degradation of methylated proteins. The methyl groups are derived from S-adenosylmethionine, with involvement of the enzymes protein arginine methyl-transferase type 1 and 2 (PRMT1, PRMT2). PRMT-1 catalyzes the formation of NG-monomethyl-L-arginine (LNMMA) and NG,NG-dimethyl-L-arginine (ADMA), while PRMT-2 methylates proteins to release NG,N'G-dimethyl-L-arginine (symmetric dimethyl-arginine; SDMA) and L-NMMA. The asymmetrically methylated arginine residues (L-NMMA and ADMA), but not symmetrically methylated arginine (SDMA), are competitive inhibitors of the nitric oxide synthases.

Nitric oxide (NO) is one of the most potent vasodilators, which is synthesized from the amino acid L-arginine. NO exerts important biological functions by stimulating guanylate cyclase to generate cGMP, inhibiting mitochondrial respiration by competing with oxygen at cytochrome oxidase, or inducing S-nitrosylation to regulate protein stability and function. The endothelium also plays a crucial role in the maintenance of vascular tone and structure. Endothelial dysfunction is known to precede overt coronary artery disease. A number of cardiovascular risk factors, as well as metabolic diseases and systemic or local inflammation cause endothelial dysfunction. Nitric oxide (NO) is one of the major endothelium-derived vasoactive substances whose role is of prime importance in maintaining endothelial homeostasis. Low levels of NO are associated with impaired endothelial function. NO production is restrained by the endogenous nitric oxide synthase (NOS) inhibitors asymmetrical dimethylarginine (ADMA) and N-monomethyl-L-arginine (L-NMMA; MMA).

In intact animals, infusion of ADMA or MMA increases vascular resistance and blood pressure. Cardiovascular diseases, including hypertension, coronary artery disease, stroke, congestive heart failure, atherosclerosis, and diabetes, are associated with increased plasma levels of ADMA with a decreased ratio of L-arginine to ADMA. Furthermore, increased plasma ADMA is a strong independent predictor of both mortality and major nonfatal cardiovascular events in patients after myocardial infarction, coronary artery disease, and stroke.

### Enzymes:

According to the present invention, the device comprises a matrix, wherein said matrix comprises an enzyme configured to metabolize ADMA and/or MMA. A skilled person is capable of assessing the activity of any given enzyme to determine whether ADMA and/or MMA are suitably metabolized. In vitro assays are available to a skilled person, for examples those described in reference [3].

For example, plasma ADMA, MMA, symmetrical dimethylarginine (SDMA), and L-arginine may be measured using a liquid chromatographic-tandem mass spectrometric method, as described in [17]. A stable isotope based technique can be employed for determination of DDAH activity, for example in plasma or tissue [18]. These techniques enable a skilled person to assess whether an enzyme shows the required metabolism of the target metabolites of the present invention.

### Dimethylarginine dimethylaminohydrolase (DDAH).

Dimethylarginine dimethylaminohydrolase is an enzyme found in all mammalian cells. Two isoforms exist, DDAH I and DDAH II, with some differences in tissue distribution of the two isoforms. The enzyme degrades methylarginines, specifically asymmetric dimethylarginine (ADMA) and monomethyl arginine (MMA).

Dimethylarginine dimethylaminohydrolase 1 (DDAH1) may be identified under RefSeq NM_012137 or UniProt O94760.

Dimethylarginine dimethylaminohydrolase 2 (DDAH2) may be identified under RefSeq NM_013974 or UniProt O95865.

Preferred sequences are presented below.

### Alanine-glyoxylate aminotransferase 2 (AGXT2):

Alanine-glyoxylate aminotransferase 2 (AGXT2) can metabolize asymmetric dimethylarginine (ADMA) via transamination to alpha-keto-delta-(NN-dimethylguanidino) valeric acid (DMGV). Unlike DDAHs, AGXT2, a mitochondrial aminotransferase expressed primarily in the kidney, can metabolize not only ADMA but also SDMA.

Alanine-glyoxylate aminotransferase 2 (AGXT2) may be identified under UniProtKB Q9BYV1 and RefSeq entries NP_001293102.1, NM_001306173.1, NP_114106.1, NM_031900.3, XP_016865237.1, XM_017009748.1.

Multiple isoforms and natural variants are known and recorded in public databases. Any given AGXT2 form capable of the enzymatic metabolism of ADMA and/or MMA as described herein is encompassed by the invention.

A preferred sequence is presented below.

### Preferred Sequences of the Invention:

DDAH1 (human)
   SEQ ID NO 1: 
MAGLGHPAAFGRATHAVVRALPESLGQHALRSAKGEEVDVARAERQHQLYVGVLGSKLGLQVV
ELPADESLPDCVFVEDVAVVCEETALITRPGAPSRRKEVDMMKEALEKLQLNIVEMKDENATLDG
GDVLFTGREFFVGLSKRTNQRGAEILADTFKDYAVSTVPVADGLHLKSFCSMAGPNLIAIGSSES
AQKALKIMQQMSDHRYDKLTVPDDIAANCIYLNIPNKGHVLLHRTPEEYPESAKVYEKLKDHMLIP
VSMSELEKVDGLLTCCSVLINKKVDS
DDAH2 (human)
   SEQ ID NO 2: 
MGTPGEGLGRCSHALIRGVPESLASGEGAGAGLPALDLAKAQREHGVLGGKLRQRLGLQLLEL
PPEESLPLGPLLGDTAVIQGDTALITRPWSPARRPEVDGVRKALQDLGLRIVEIGDENATLDGTDV
LFTGREFFVGLSKWTNHRGAEIVADTFRDFAVSTVPVSGPSHLRGLCGMGGPRTVVAGSSDAA
QKAVRAMAVLTDHPYASLTLPDDAAADCLFLRPGLPGVPPFLLHRGGGDLPNSQEALQKLSDVT
LVPVSCSELEKAGAGLSSLCLVLSTRPHS
AGXT2 (human)
   SEQ ID NO 3:

MTLIWRHLLRPLCLVTSAPRILEMHPFLSLGTSRTSVTKLSLHTKPRMPPCDFMPERYQSLGYNR
VLEIHKEHLSPVVTAYFQKPLLLHQGHMEWLFDAEGSRYLDFFSGIVTVSVGHCHPKVNAVAQK
QLGRLWHTSTVFFHPPMHEYAEKLAALLPEPLKVIFLVNSGSEANELAMLMARAHSNNIDIISFRG

AYHGCSPYTLGLTNVGTYKMELPGGTGCQPTMCPDVFRGPWGGSHCRDSPVQTIRKCSCAPD
CCQAKDQYIEQFKDTLSTSVAKSIAGFFAEPIQGVNGVVQYPKGFLKEAFELVRARGGVCIADEV
QTGFGRLGSHFWGFQTHDVLPDIVTMAKGIGNGFPMAAVITTPEIAKSLAKCLQHFNTFGGNPM
ACAIGSAVLEVIKEENLQENSQEVGTYMLLKFAKLRDEFEIVGDVRGKGLMIGIEMVQDKISCRPL
PREEVNQIHEDCKHMGLLVGRGSIFSQTFRIAPSMCITKPEVDFAVEVFRSALTQHMERRAK

In some embodiments, a DDAH1, DDAH2 orAGXT2 protein with substantially the same or a similar amino acid sequence to SEQ ID NO 1-3, or fragments thereof, may be employed. Examples are fragments of a DDAH1, DDAH2 orAGXT2 protein, such as naturally occurring fragments, homologues or derivatives with essentially the same properties or functional analogy to the examples of a DDAH1, DDAH2 or AGXT2 protein as described herein.

As used herein, the term "substantially the same or similar amino acid sequence" includes an amino acid sequence that is similar, but not identical to, the reference amino acid sequence, such as in SEQ ID NO 1-3. For example, an amino acid sequence, i.e., polypeptide, that has substantially the same amino acid sequence as SEQ ID NO 1-3, and comprises one or more modifications, such as amino acid additions, deletions, or substitutions relative to the amino acid sequence of SEQ ID NO 1-3, may be employed, provided that the modified polypeptide retains substantially at least one biological activity such as those described above, i.e. the desired metabolism of ADMA and/or MMA in the blood or plasma of a patient.

A particularly useful modification of a polypeptide of the present invention, or a fragment thereof, is a modification that confers, for example, increased stability or reactivity. Incorporation of one or more D-amino acids is a modification useful in increasing stability of a polypeptide or polypeptide fragment. Similarly, deletion or substitution of lysine residues can increase stability by protecting the polypeptide or polypeptide fragment against degradation.

The amino acid sequences may also comprise 0 to 100, 2 to 50, 5 to 20, or for example 8 to 15, or any value from 0 to 20, amino acid additions or deletions at either the N- and/or C-terminus of the proteins. The termini may also be modified with additional linker sequences, or removal of sequences, as long as the properties of the protein are essentially maintained.

Various ways of preparing functionally analogous peptides have been disclosed in the prior art. Peptides designed starting from the peptides of the invention using such methods are included in the teaching according to the invention. For example, one way of generating functionally analogous peptides has been described in PNAS USA 1998, Oct. 13, 95(21), 12179-84; WO 02/38592; the above teachings are hereby incorporated in the disclosure of the invention. That is, all peptides, peptide fragments or structures comprising peptides generated using the methods mentioned above - starting from the peptides of the invention - are peptides according to the invention, provided they accomplish the object of the invention.

### Diseases to be treated:

According to the present invention, essentially any medical condition associated with elevated ADMA and/or MMA levels compared to healthy controls, is a target indication for treatment with the blood treatment device of the present invention,

### Vascular and/or cardiac disease:

A number of studies have shown a relationship between raised ADMA concentrations and cardiovascular disease. Raised plasma ADMA concentrations have been reported in people with coronary artery disease (CAD), peripheral arterial disease, chronic heart failure, pulmonary hypertension, preeclampsia, stroke and hypertrophic cardiomyopathy.

Vascular and/or cardiac disease are, without limitation, preferably selected from the group consisting of coronary arterial disease, peripheral atrial disease, cerebral arterial disease, heart failure, dyslipidemias, arterial hypertension, pulmonary hypertension, eclampsia, digital ulcera, erectile dysfunction, migraines, traumatic vascular injuries, transplant vasculopathies. microvascular injuries, myocardial remodeling and vascular remodeling, ischemia-reperfusion injuries and/or vasospastic diseases, or other vascular or cardiac disease associated with ADMA and/or MMA.

### Vascular and cardiac complications of metabolic diseases:

Vascular and cardia complications of metabolic diseases can arise and are often associated with elevated ADMA and/or MMA levels. Metabolic disease is considered any of the diseases or disorders that disrupt normal metabolism. Examples of such conditions are selected from such as diabetes mellitus, preferably type 2, dyslipidemias, hyperhomocysteinemia and/or hyperuricemia. Cardiovascular disease is often grouped with metabolic disorders, or is an associated complication of metabolic diseases, because it is frequently a consequence of diabetes and dyslipidemia.

### Hepatic and/or renal disease:

Nitric oxide (NO) synthesis is blocked by asymmetric dimethylarginine (ADMA) which competes with L-arginine for NO-synthase. ADMA is metabolized principally in the liver, by dimethylarginine dimethylaminohydrolase. The kidney and the liver are involved in ADMA excretion. A correlation exists between plasma ADMA levels and degree of hepatic dysfunction in patients suffering from liver cirrhosis, alcoholic hepatitis and acute liver failure. High plasma ADMA levels are relevant because they are associated with the development of multi-organ failure (MOF). Increased plasma ADMA concentration was identified as a risk factor for MOF in critically-ill patients causing enhanced Intensive Care Unit mortality.

Since ADMA is a uremic toxin, the pathophysiological relevance of ADMA has been extensively investigated in chronic kidney disease (CKD) and end-stage renal disease (ESRD). Although ADMA has been shown to correlate with cardiovascular risk factors, its plasma concentration also increases in patients suffering from end-stage kidney disease and hepatic dysfunction. In particular, a significant correlation has been demonstrated between plasma ADMA levels and the degree of hepatic dysfunction in patients suffering from liver diseases with varying aetiologies.

Notably, plasma ADMA levels are increased in patients with liver cirrhosis, alcoholic hepatitis and acute liver failure, all of which could be addressed by the present application.

The mechanism by which liver dysfunction results in raised ADMA concentrations is probably due to impaired DDAH activity, which is highly expressed in normal livers: local processes such as severe inflammation, oxidative stress, and direct damage to DDAH protein may underlie a significant deterioration in DDAH activity in critically ill patients with hepatic dysfunction leading to elevation of ADMA concentrations.

### Further indications:

Putatively, a number of risk factors, including obesity, hypertension, hypercholesterolemia, smoking, diabetes mellitus, hyperhomocysteinemia and vascular inflammation potentially mediate their deleterious effects on the vascular wall through the dysfunction of the endothelial L-arginine/NO pathway. In each of these conditions, ADMA plasma levels have been shown in case-control studies to be elevated compared to healthy controls.

### Devices and immobilization of the enzyme in the device:

The "matrix" as used herein thus refers to a material inside the blood treatment device that provides an internal material or surface through or over which blood or plasma is passed. The matrix as used in the context of the present invention preferably comprises a support to which the enzyme is bound. The support therefore serves as a carrier for the enzyme, even though it may fulfil other functions.

The "support" as used herein refers to the portion of the matrix which serves as the "substrate" or "support material" to which the enzymes according to the invention are bound. Such support or support material is sometimes also referred to as "adsorption material" or "adsorber", as used in an "adsorption column" or "column" or "adsorption cartridge". A suitable support according to the present invention should be uniform, hydrophilic, mechanically and chemically stable over the relevant pH range and temperature with no or a negligible leaching of the enzyme during use, have good flow characteristics for whole blood and/or blood plasma, and provides a large surface area for enzyme attachment.

The support can for example be a resin, a membrane or a non-woven material. A "non-woven" material refers to a material which is broadly defined as a sheet, fabric or web structure bonded together by entangling fiber or filaments (and by perforating films) mechanically, thermally, or chemically but not by weaving or knitting. A "resin" refers to an insoluble material which can take the form of gels or gel beads or microporous beads, or a sponge. Such resins can be natural or bio-polymers, synthetic polymers and inorganic materials. Agarose, dextrose and cellulose beads are commonly employed natural supports. Synthetic polymeric or organic supports are mostly based on acrylamide, polystyrene and polymethacrylate derivatives, whereas, porous silica and glass are some frequently used inorganic supports.

According to one embodiment of the invention, the resin is composed of polymers selected from the group consisting of alginate, chitosan, chitin, collagen, carrageenan, gelatin, cellulose, starch, pectin and sepharose; inorganic materials selected from the group consisting of zeolites, ceramics, celite, silica, glass, activated carbon and char-coal; or synthetic polymers selected from the group consisting of polyethylene (PE), polyoxymethylene (POM), polypropylene (PP), polyvinylchloride (PVC), polyvinyl acetate (PVA), polyvinylidene chloride (PVDC), polystyrene (PS), polytetrafluoroethylene (PTFE), polyacrylate (PAA), polymethyl methacrylate (PMMA), polyacrylamide, polyglycidyl methac-rylate (PGMA), acrylonitrile butadiene styrene (ABS), polyacrylonitrile (PAN), polyester, polycarbonate, polyethylene terephthalate (PET), polyamide, polyaramide, polyethylene glycol (PEG), polyvinylpyrrolidone (PVP), polysulfone (PS), polyethersulfone (PES), polyarylethersulfone (PEAS), eth-ylene vinyl acetate (EVA), ethylene vinyl alcohol (EVOH), polyamideimide, polyaryletherketone (PAEK), polybutadiene (PBD), polybutylene (PB), polybutylene terephthalate (PBT), polycaprolactone (PCL), polyhydroxyalkanoate, polyether ether ketone (PEEK), polyether ketone ketone (PEKK), polyether imide (PEI), polyimide, polylactic acid (PLA), polymethyl pentene (PMP), poly(p-phenylene ether) (PPE), polyurethane (PU), styrene acrylonitrile (SAN), polybutenoic acid, poly(4-allyl-benzoic acid), poly(glycidyl acrylate), polyglycidyl methacry-late (PGMA), acrylonitrile butadiene styrene (ABS), polydivinylbenzene (PDVB), poly(allyl glycidyl ether), poly(vinyl glycidyl ether), poly(vinyl glycidyl urethane), polyallylamine, pol-yvinylamine, copolymers of said polymers and any of these polymers modified by introduction of functional groups.

Various known methods can be used to immobilize the enzyme to the support and/or matrix according to the invention. Such immobilization preferably is specific or selective in that it immobilizes the enzyme whereas other proteins and components present in blood or blood plasma or a sample thereof (*in vitro*) are not immobilized to a significant degree.

The "immobilizing" of an enzyme to the support for providing a matrix which can be used in a device according to the invention refers to a non-covalent or covalent interaction that holds two molecules together. According to one embodiment of the invention, the expression refers to a covalent interaction, i.e. to covalently bound enzymes. Non-covalent interactions include, but are not limited to, hydrogen bonding, ionic interactions among charged groups, van der Waals interactions, and hydrophobic interactions among non-polar groups. One or more of these interactions can mediate the binding of two molecules to each other. Binding may otherwise be specific or selective, or unspecific.

According to one embodiment, the enzyme comprises affinity tags for immobilizing it on the support. Affinity tags can be used for purifying the protein during production and/or for immobilizing them on the support of the matrix of the present invention. Affinity tags can be short polypeptide sequences or whole proteins, co-expressed as fusion partners with the enzymes. Different types of affinity tags are well known in the art, wherein polyhistidine or Hiss-tags, C-myc-tags and FLAG-tags are especially well described and are options for binding the enzymes according to the invention to the support material. The noncovalent linkage of biotin to strepavidin or avidin can also be used to immobilize the enzyme to a support.

According to another embodiment of the invention, the enzymes are covalently attached to the support as further detailed below and/or as described the prior art. Covalent coupling generally includes either covalent non-site directed attachment of the protein or site-directed attachment of the protein. The support which forms the basis for the generation of a matrix must provide or facilitate chemical activation, thus allowing the chemical coupling of the enzyme. Many coupling methods for immobilizing enzymes are well known in the art.

For example, the activation chemistry should be stable over a wide range of pH, buffer conditions and temperature resulting in negligible leaching of the enzymes. The coupling method should avoid improper orientation, multisite attachment or steric hindrance of the enzyme. The enzyme density per volume of matrix can be optimized to promote target accessibility and reaction.

The covalent coupling can be carried out via common functional groups, including amines, alcohols, carboxylic acids, aldehydes and epoxy groups. Carbodiimide compounds can be used to activate carboxylic groups of proteins for direct conjugation to the primary amines on the support surface via amide bonds. The most commonly used carbodiimides are the water-soluble EDC (1-ethyl-3-(-3-dimethylaminopropyl) carbodiimide) for aqueous crosslinking and the water-insoluble DCC (N', N'-dicyclohexyl carbodiimide) for non-aqueous organic synthesis methods.

Alternatively, the supports may carry specific functional groups for coupling a linker and/or enzyme thereto. For example, functionalized resins are commercially available and known to a person with skill in the art. A wide range of coupling chemistries, involving primary amines, sulfhydryls, aldehydes, hydroxyls and carboxylic acids are available in said commercial supports. Examples for commercially available activated resins are CarboLink Coupling resin, Profinity^{™} Epoxide resin, Affi-Gel 10 and 15, Epoxy-activated Sepharose^{™} 6B, Tresyl chloride-activated agarose, and Purolite^{®} Lifetech^{™} methacrylate polymers functionalized with epoxy groups.

According to one embodiment of the invention, the support material should be porous, wherein the pore size is in the range of from 10 to 200 nm. According to another embodiment of the invention, the support takes the form of beads. According to yet another embodiment, the support according to the invention comprises magnetic beads. Magnetic beads are prepared by entrapping magnetite within agarose or other polymeric material, on which the enzyme according to the invention is immobilized.

According to another embodiment of the present invention the support is a membrane. Membranes as components of affinity matrices have been used in protein purification, due to their simplicity, ease of handling, reduced surface area and lower diffusion limitations compared to gels, resins and beads. The membranes can take the physical form of a hollow fiber or, alternatively, of a flat sheet membrane. According to one embodiment, the support comprises a hemodialysis hollow fiber membrane dialyzer, wherein the filter is a hemodialyzer.

The hollow fiber or flat sheet membranes for use as supports in a device according to the invention may be composed of cellulose, cellulose ester (cellulose acetate and cellulose triacetate), poly(methylmethacrylate)(PMMA), polyamide (PA), other nitrogen-containing polymers (polybenzimidazole, polyacrylonitrile (PAN), polyglycidyl methacrylate (PGMA), polyvinylpyrrolidone (PVP), polysulfone (PS), polyethersulfone (PES) or polyarylethersulfone (PAES). A hollow fiber membrane which can advantageously be utilized for providing a device according to the invention preferably has an inner diameter in the range of 100 to 500 µm. According to another embodiment of the invention, specifically when the membrane support is a hemodialysis membrane as described above, the hollow fiber membranes are additionally or alternatively functionalized with an enzyme according to the invention on the lumen side of the fibers where they can directly interact with the target metabolite in the blood or blood plasma which perfuses the lumen of the hollow fiber membrane. The enzyme may also or alternatively be immobilized to the outside of the membrane.

### Methods of extracorporeal blood treatment:

The invention includes devices which are configured to be located in an extracorporeal blood circuit through which the blood of a patient passes and which comprises means for transporting blood from the patient's vascular system to a blood treatment device at a defined flow rate and then returning the treated blood back to the patient, and wherein the device is further configured to reduce levels of ADMA and/or MMA in the blood.

According to the invention, the expression "extracorporeal blood purification" refers preferably to the process of removing substances from body fluids through their clearance from flowing blood in a diverted circuit outside the patient's body (extracorporeal). Said substances may include endogenous toxins (i.e., uremic toxins), exogenous poisons (i.e., ethylene glycol or fungal toxin), administered drugs, viruses, bacteria, antibodies, metabolites and proteins (i.e., IMHA, myasthenia gravis), abnormal cells (i.e., leukemia), and excessive water. Therapeutic procedures include hemodialysis, including intermittent hemodialysis (HD, HDF, HF) and continuous renal replacement therapy (CRRT); hemoperfusion; plasma exchange and therapeutic apheresis. Such methods are known to a skilled person and the device of the invention can be incorporated accordingly.

The expression "blood" as used herein refers to whole blood which contains all components of the blood of an organism, including red cells, white cells, and platelets suspended in plasma. The expression "blood plasma" refers to the fluid, composed of about 92% water, 7% proteins such as albumin, gamma globulin, fibrinogen, complement factors, clotting factors, and 1% mineral salts, sugars, fats, electrolytes, hormones and vitamins which forms part of whole blood but no longer contains red and white cells and platelets. In the context of the present invention, the expression "blood plasma" or "plasma" refers to specific fractions of the above defined blood plasma in its standard meaning, such as, for example, blood serum.

According to one aspect, blood flow rates in an extracorporeal blood purification circuit are between 20 ml and 700 ml/min. Typical dialysate flow rates in an extracorporeal circuit comprising a hemodialyzer for the treatment of renal failure, either in addition to the blood treatment device according to the invention or in cases where the hemodialyzer in addition is configured to metabolie ADMA and/or MMA, is in the range of between 0,5 l/h and 800 ml/min.

In therapeutic apheresis whole blood can be treated or blood is separated into its component fractions, for example by centrifugation or by means of a plasma membrane or filter, and the fraction containing the solute which shall be removed, is specifically treated prior to return to the patient.

The present invention provides for an apheresis treatment in which whole blood or plasma (containing the target proteins) is removed from the patient's flowing blood and, after having been contacted with a device or matrix according to the invention is returned to the patient. Typical blood or plasma flow rates in an extracorporeal circuit wherein the blood treatment device is perfused with whole blood or plasma is in the range of between 30 ml/min and 200 ml/min, or 7 ml/min and 50 ml/min respectively.

According to one aspect, the extracorporeal blood circuit according to the invention is configured to perform hemodialysis. In this case, the device according to the invention is, for example, a hemodialyzer which additionally has been configured to immobilize a target protein according to the invention. The circuit can be operated in different treatment modes depending on the medical need, including hemodialysis, hemodiafiltration, hemofiltration mode.

### FIGURES

The invention is further described by the following figures. There are intended to represent a more detailed illustration of a number of preferred non-limiting embodiments or aspects of the invention without limiting the scope of the invention described herein.

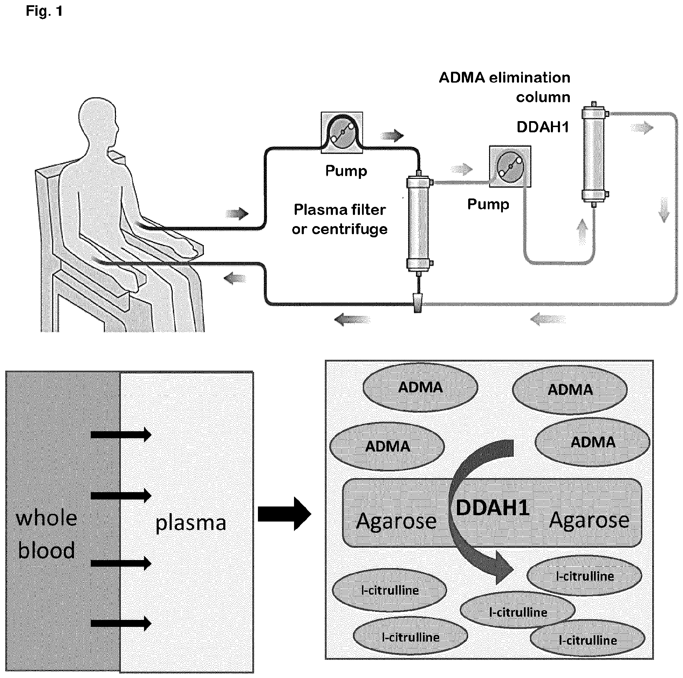

### Figure 1: Schematic outline of a preferred embodiment of the invention.

Blood is drawn from the patient through a venous access, anticoagulated and separated into its liquid and cellular components using a centrifuge or a filter. The separated plasma is perfused through an ADMA elimination column, as described herein, containing active DDAH1, DDAH2 and/or AGXT2 molecules (shown in the figure is the preferred DDAH1), bound on the column matrix (preferably agarose gel) that results in metabolism of ADMA and MMA, preferably via catabolism of ADMA to I-citrulline. The depleted plasma is reunited with the cellular blood constituents and returned to the patient via the blood return line.

### EXAMPLES

The invention is further described by the following examples. These are intended to present support for the workability of a number of preferred non-limiting embodiments or aspects of the invention without limiting the scope of the invention described herein.

### Example 1: In vitro trial

Recombinant DDAH-1 and/or-2 protein is coupled to agarose beads. The activity of immobilized DDAH enzyme for catabolism of ADMA and MMA is tested by incubating human plasma with the DDAH-beads in vitro for varying lengths of time and by subsequently measuring the concentration of L-citrulline as the product of ADMA and/or MMA hydrolytic degradation by DDAH. L-citrulline measurements from plasma samples are conducted using appropriate commercially available kits (e.g. L-citrulline ELISA kits, ALPCO 30-6600, colormetric assays, Immundiagnostik AG K6600).

Initial experiments demonstrate a high DDAH activity in vitro after coupling to agarose beads. This result represents a significant precondition for the development of a column with an immobilized enzyme for extracorporeal ADMA and/or MMA elimination.

### Example 2: Production of the ADMA column

For the depletion of ADMA, recombinant DDAH-1 and/or -2 is coupled to an agarose resin following an established protocol. The resin is first flushed and equilibrated. The coupling solution containing DDAH protein is added to the equilibrated agarose. The mixture is gently shaken for several hours. After coupling, the resin is washed several times and resuspended in phosphate-buffered saline (PBS). For testing of the resin, the resin is filled into commercially available plastic columns.

### Example 3: Animal model

ADMA column experiments to show DDAH protein activity after resin-coupling using plasma samples are carried out using appropriate mini pig models with induced myocardial infarction. Suitable models are established in the art (e.g. Schuleri et al, Comp Med. 2008 Dec;58(6):568-79, Mizuno et al, Eur J Pharmacol. 2018 834:103-108, Hirano et al, Translational Medicine Communications 2017 2:1). The scar size and cardiac performance before, immediately and in 24 hours after the infarction onset are measured. Measurements are carried out in plasma samples obtained from multiple minipigs with induced myocardial infarction either with or without treatment using DDAH-columns.

### Example 4: Clinical trials

The first trials are planned in patients with acute ST- elevated myocardial infarction (STEMI), who have stable hemodynamics and no arrhythmias in the intensive care unit. The commercially available therapeutic apheresis system (ADAsorb^{®}) will be used. Between 3000-6000 ml plasma will be treated with an ADMA column via peripheral venous access. Dependent on the ADMA reduction value, the apheresis sessions will be performed from 1 to 3 times within the first 72 hours after the patient's admission, taking into consideration all conventional procedures (PTA, stenting etc.). The measurements of laboratory myocardial damage markers, electrocardiography and echocardiography will be performed according to conventional guidelines. The scar size will be determined by MRI using established techniques within the first 6 hours and in two days after the patient admission in the hospital. In control patients a sham apheresis will be performed.

### References:

1. Boger RH. Asymmetric dimethylarginine, an endogenous inhibitor of nitric oxide synthase, explains the "l-arginine paradox" and acts as a novel cardiovascular risk factor. J Nutr. 2004;134:2842S-2847S; discussion 2853S
2. Leiper J, Nandi M, Torondel B, Murray-Rust J, Malaki M, O'Hara B, Rossiter S, Anthony S, Madhani M, Selwood D, Smith C, Wojciak-Stothard B, Rudiger A, Stidwill R, McDonald NQ, Vallance P. Disruption of methylarginine metabolism impairs vascular homeostasis. Nature medicine. 2007;13:198-203
3. Hu X, Atzler D, Xu X, Zhang P, Guo H, Lu Z, Fassett J, Schwedhelm E, Boger RH, Bache RJ, Chen Y. Dimethylarginine dimethylaminohydrolase-1 is the critical enzyme for degrading the cardiovascular risk factor asymmetrical dimethylarginine. Arteriosclerosis, thrombosis, and vascular biology. 2011;31:1540-1546
4. Dayoub H, Rodionov RN, Lynch C, Cooke JP, Arning E, Bottiglieri T, Lentz SR, Faraci FM. Overexpression of dimethylarginine dimethylaminohydrolase inhibits asymmetric dimethylarginine-induced endothelial dysfunction in the cerebral circulation. Stroke. 2008;39:180-184
5. Jacobi J, Sydow K, von Degenfeld G, Zhang Y, Dayoub H, Wang B, Patterson AJ, Kimoto M, Blau HM, Cooke JP. Overexpression of dimethylarginine dimethylaminohydrolase reduces tissue asymmetric dimethylarginine levels and enhances angiogenesis. Circulation. 2005;111:1431-1438
6. Sydow K, Mondon CE, Schrader J, Konishi H, Cooke JP. Dimethylarginine dimethylaminohydrolase overexpression enhances insulin sensitivity. Arteriosclerosis, thrombosis, and vascular biology. 2008;28:692-697
7. Stuhlinger MC, Conci E, Haubner BJ, Stocker EM, Schwaighofer J, Coöke JP, Tsao PS, Pachinger 0, Metzler B. Asymmetric dimethyl l-arginine (adma) is a critical regulator of myocardial reperfusion injury. Cardiovascular research. 2007;75:417-425
8. Nakayama Y, Ueda S, Yamagishi S, Obara N, Taguchi K, Ando R, Kaida Y, Iwatani R, Kaifu K, Yokoro M, Toyonaga M, Kusumoto T, Fukami K, Okuda S. Asymmetric dimethylarginine accumulates in the kidney during ischemia/reperfusion injury. Kidney international. 2014;85:570-578
9. Jacobi J, Maas R, Cardounel AJ, Arend M, Pope AJ, Cordasic N, Heusinger-Ribeiro J, Atzler D, Strobel J, Schwedhelm E, Boger RH, Hilgers KF. Dimethylarginine dimethylaminohydrolase overexpression ameliorates atherosclerosis in apolipoprotein e-deficient mice by lowering asymmetric dimethylarginine. The American journal of pathology. 2010;176:2559-2570
10. Rodionov RN, Dayoub H, Lynch CM, Wilson KM, Stevens JW, Murry DJ, Kimoto M, Arning E, Bottiglieri T, Cooke JP, Baumbach GL, Faraci FM, Lentz SR. Overexpression of dimethylarginine dimethylaminohydrolase protects against cerebral vascular effects of hyperhomocysteinemia. Circulation research. 2010;106:551-558
11. Sydow K, Schmitz C, von Leitner EC, von Leitner R, Klinke A, Atzler D, Krebs C, Wieboldt H, Ehmke H, Schwedhelm E, Meinertz T, Blankenberg S, Boger RH, Magnus T, Baldus S, Wenzel U. Dimethylarginine dimethylaminohydrolasel is an organ-specific mediator of end organ damage in a murine model of hypertension. PLoS One. 2012;7:e48150
12. Kinan Rifai, Stefanie M. Bode-Boeger, Jens Martens-Lobenhoffer, Thomas Ernst, Ulrich Kretschmer, Carsten Hafer, Danilo Fliser, Michael Peter Manns & Jan T. Kielstein, Removal of asymmetric dimethylarginine during artificial liver support using fractionated plasma separation and adsorption. Scandinavian Journal of Gastroenterology, 2010; Early Online, 1-6
13. Ding H, Wu B, Wang H, Lu Z, Yan J, Wang X, Shaffer JR, Hui R, Wang DW. A novel loss-of-function DDAH1 promoter polymorphism is associated with increased susceptibility to thrombosis stroke and coronary heart disease. Circ Res. 2010;106:1145-1152.
14. Leiper JM, Santa Maria J, Chubb A, MacAllister RJ, Charles IG, Whitley GS, Vallance P. Identification of two human dimethylarginine dimethylaminohydrolases with distinct tissue distributions and homology with microbial arginine deiminases. Biochem J. 1999;343:209-214.
15. Rodionov R.N., Murry D.J., Vaulman S.F., Stevens J.W., Lentz S.R. Human alanine-glyoxylate aminotransferase 2 lowers asymmetric dimethylarginine and protects from inhibition of nitric oxide production. J. Biol. Chem. 285:5385-5391(2010)
16. Caplin B., Wang Z., Slaviero A., Tomlinson J., Dowsett L., Delahaye M., Salama A., Wheeler D.C., Leiper J. Alanine-Glyoxylate aminotransferase-2 metabolizes endogenous methylarginines, regulates NO, and controls blood pressure. Arterioscler. Thromb. Vasc. Biol. 32:2892-2900(2012)
17. Schwedhelm E, Maas R, Tan-Andresen J, Schulze F, Riederer U, Böger RH. High-throughput liquid chromatographic-tandem mass spectrometric determination of arginine and dimethylated arginine derivatives in human and mouse plasma. J Chromatogr B Analyt Technol Biomed Life Sci. 2007;851:211-219.
18. Maas R, Tan-Andreesen J, Schwedhelm E, Schulze F, Böger RH. A stable-isotope based technique for the determination of dimethylarginine dimethylaminohydrolase (DDAH) activity in mouse tissue. J Chromatogr B Analyt Technol Biomed Life Sci. 2007;851:220-228.

## Claims

1. A blood treatment device configured to lower the amount of asymmetric dimethylarginine (ADMA) and/or monomethyl arginine (MMA) in the blood or blood plasma of a person in need thereof in an extracorporeal blood circuit, wherein the device comprises a matrix, and wherein said matrix comprises dimethylarginine dimethylaminohydrolase (DDAH) and/or alanine-glyoxylate aminotransferase 2 (AGXT2).

2. A blood treatment device according to claim 1, wherein:
a. the dimethylarginine dimethylaminohydrolase (DDAH) is immobilized to the matrix and configured to hydrolyze asymmetric dimethylarginine (ADMA) and/or monomethyl arginine (MMA) by catabolism of ADMA and/or MMA to L-citrulline in blood or blood plasma, and/or
b. the alanine-glyoxylate aminotransferase 2 (AGXT2) is immobilized to the matrix and configured to metabolize asymmetric dimethylarginine (ADMA) and/or monomethyl arginine (MMA) via transamination to alpha-keto-delta-(NN-dimethylguanidino) valeric acid (ADGV) and alpha-keto-delta-(N-monomethylguanidino) valeric acid (MGV), respectively, in blood or blood plasma.

3. A blood treatment device according to any one of the preceding claims, wherein the dimethylarginine dimethylaminohydrolase (DDAH) comprises or consists of isoform 1 (DDAH1) and/or isoform 2 (DDAH2).

4. A blood treatment device according to any one of the preceding claims, wherein the matrix comprises a support to which dimethylarginine dimethylaminohydrolase (DDAH) and/or alanine-glyoxylate aminotransferase 2 (AGXT2) is bound, wherein the support comprises or consists of a material selected from the group consisting of a resin, bead, fiber mat, non-woven material, porous foam and membrane, such as a tubular, hollow-fiber or flat sheet membrane.

5. A blood treatment device according to the preceding claim, wherein the support and/or matrix comprises or consists of at least one polymer, preferably selected from the group consisting of alginate, chitosan, chitin, collagen, carrageenan, gelatin, starch, pectin, cellulose, agarose, dextrose and sepharose; an inorganic material, preferably selected from the group consisting of ceramics, celite, silica, glass, activated carbon and charcoal; or a synthetic polymer.

6. A blood treatment device according to any one of the preceding claims, wherein dimethylarginine dimethylaminohydrolase (DDAH) and/or alanine-glyoxylate aminotransferase 2 (AGXT2) is covalently bound to the matrix, such as via cross-linking, or is noncovalently bound to the matrix, such as via ionic interactions or an affinity tag.

7. A blood treatment device according to any one of the preceding claims, wherein the blood treatment device is located in an extracorporeal blood circuit through which the blood of the patient passes, wherein said circuit comprises means for transporting blood from the patient's vascular system to the blood treatment device at a defined flow rate and for returning the treated blood back to the patient.

8. A blood treatment device according to the preceding claim, wherein the extracorporeal blood circuit in which the blood treatment device is located further comprises a plasma separator, such as a plasma filter or centrifuge-based plasma separation system or combination thereof (for example a disk separator), which allows for the separation of plasma from the blood, and wherein the blood treatment device is located downstream of a plasma outlet of the plasma separator.

9. A blood treatment device according to claim 7, wherein the extracorporeal blood circuit in which the blood treatment device is located further comprises a hemofilter for the dialysis of blood which is located upstream or downstream of the blood treatment device, or wherein the blood treatment device is a hemofilter for the dialysis of blood.

10. A blood treatment device according to claims 7 or 9, wherein the blood treatment device is a column and is perfused with whole blood.

11. An extracorporeal blood circuit comprising a blood treatment device according to any one of claims 1 to 10, wherein the extracorporeal blood circuit comprises means for transporting blood or blood plasma from the patient's vascular system to the blood treatment device at a defined flow rate and means for returning the treated blood or blood plasma back to the patient.

12. A blood treatment device according to any one of the preceding claims for use as a medicament in the treatment and/or prevention of:
a. a medical condition associated with elevated asymmetric dimethylarginine (ADMA) and/or monomethyl arginine (MMA) levels in the blood or blood plasma of a subject, compared to healthy controls, and/or
b. a medical condition associated with impaired nitric oxide (NO) production and/or bioavailability compared to healthy controls.

13. The blood treatment device for use as a medicament according to claim 12, wherein the medical condition is:
a. a vascular and/or cardiac disease, preferably selected from the group consisting of coronary arterial disease, peripheral atrial disease, cerebral arterial disease, heart failure, dyslipidemias, arterial hypertension, pulmonary hypertension, eclampsia, digital ulcera, erectile dysfunction, migraines, traumatic vascular injuries, transplant vasculopathies. microvascular injuries, myocardial remodeling and vascular remodeling, ischemia-reperfusion injuries and/or vasospastic diseases.
b. vascular and cardiac complications of metabolic diseases, such as diabetes mellitus, dyslipidemias, hyperhomocysteinemia and/or hyperuricemia
c. a hepatic and/or renal disease

14. An enzyme that metabolizes asymmetric dimethylarginine (ADMA) and/or monomethyl arginine (MMA), selected from dimethylarginine dimethylaminohydrolase (DDAH) and/or alanine-glyoxylate aminotransferase 2 (AGXT2), for use as a medicament in the treatment and/or prevention of a medical condition associated with elevated asymmetric dimethylarginine (ADMA) and/or monomethyl arginine (MMA) levels in the blood or blood plasma of a subject, compared to healthy controls, wherein said enzyme is immobilized to a matrix of a blood treatment device.

## Patentansprüche

1. Blutbehandlungsvorrichtung, die konfiguriert ist, um die Menge an asymmetrischem Dimethylarginin (ADMA) und/oder Monomethylarginin (MMA) im Blut oder Blutplasma einer Person, die dessen bedarf, in einem extrakorporalen Blutkreislauf zu senken, wobei die Vorrichtung eine Matrix umfasst und wobei die Matrix Dimethylarginin-Dimethylaminohydrolase (DDAH) und/oder Alanin-Glyoxylat-Aminotransferase 2 (AGXT2) umfasst.

2. Blutbehandlungsvorrichtung nach Anspruch 1, wobei:
a. die Dimethylarginin-Dimethylaminohydrolase (DDAH) an der Matrix immobilisiert und konfiguriert ist, um asymmetrisches Dimethylarginin (ADMA) und/oder Monomethylarginin (MMA) durch Katabolismus von ADMA und/oder MMA zu L-Citrullin in Blut oder Blutplasma zu hydrolysieren, und/oder
b. die Alanin-Glyoxylat-Aminotransferase 2 (AGXT2) an der Matrix immobilisiert und konfiguriert ist, um asymmetrisches Dimethylarginin (ADMA) und/oder Monomethylarginin (MMA) durch Transaminierung zu Alpha-keto-delta-(NN-dimethylguanidino)valeriansäure (ADGV) bzw. Alpha-keto-delta-(N-monomethylguanidino)valeriansäure (MGV) in Blut oder Blutplasma zu metabolisieren.

3. Blutbehandlungsvorrichtung nach einem der vorhergehenden Ansprüche, wobei die Dimethylarginin-Dimethylaminohydrolase (DDAH) Isoform 1 (DDAH1) und/oder Isoform 2 (DDAH2) umfasst oder daraus besteht.

4. Blutbehandlungsvorrichtung nach einem der vorhergehenden Ansprüche, wobei die Matrix einen Träger umfasst, an den Dimethylarginin-Dimethylaminohydrolase (DDAH) und/oder Alanin-Glyoxylat-Aminotransferase 2 (AGXT2) gebunden ist, wobei der Träger ein Material ausgewählt aus der Gruppe, bestehend aus einem Harz, einem Kügelchen, einer Fasermatte, einem Vliesmaterial, einem porösem Schaum und einer Membran, wie einer röhrenförmigen, Hohlfaser- oder flachlagigen Membran, umfasst oder daraus besteht.

5. Blutbehandlungsvorrichtung nach dem vorhergehenden Anspruch, wobei der Träger und/oder die Matrix mindestens ein Polymer, bevorzugt ausgewählt aus der Gruppe, bestehend aus Alginat, Chitosan, Chitin, Kollagen, Carrageenan, Gelatine, Stärke, Pektin, Cellulose, Agarose, Dextrose und Sepharose; ein anorganisches Material, bevorzugt ausgewählt aus der Gruppe bestehend aus Keramik, Kieselgur, Kieselsäure, Glas, Aktivkohle und Holzkohle; oder ein synthetisches Polymer umfasst oder daraus besteht.

6. Blutbehandlungsvorrichtung nach einem der vorhergehenden Ansprüche, wobei Dimethylarginin-Dimethylaminohydrolase (DDAH) und/oder Alanin-Glyoxylat-Aminotransferase 2 (AGXT2) kovalent an die Matrix gebunden ist, wie über Vernetzung, oder nichtkovalent an die Matrix gebunden ist, wie etwa über ionische Wechselwirkungen oder eine Affinitätsmarkierung.

7. Blutbehandlungsvorrichtung nach einem der vorhergehenden Ansprüche, wobei sich die Blutbehandlungsvorrichtung in einem extrakorporalen Blutkreislauf befindet, durch den das Blut des Patienten fließt, wobei der Kreislauf Mittel zum Transportieren von Blut von dem Gefäßsystem des Patienten zu der Blutbehandlungsvorrichtung mit einer definierten Flussrate und zum Rückführen des behandelten Blutes zurück zu dem Patienten umfasst.

8. Blutbehandlungsvorrichtung nach einem der vorhergehenden Ansprüche, wobei der extrakorporale Blutkreislauf, in dem sich die Blutbehandlungsvorrichtung befindet, ferner einen Plasmaseparator, wie ein Plasmafilter oder ein zentrifugenbasiertes Plasmaseparationssystem oder eine Kombination davon (beispielsweise einen Scheibenseparator), umfasst, der die Separation von Plasma aus dem Blut ermöglicht, und wobei sich die Blutbehandlungsvorrichtung stromabwärts eines Plasmaauslasses des Plasmaseparators befindet.

9. Blutbehandlungsvorrichtung nach Anspruch 7, wobei der extrakorporale Blutkreislauf, in dem sich die Blutbehandlungsvorrichtung befindet, ferner ein Hämofilter zur Dialyse von Blut umfasst, das sich stromaufwärts oder stromabwärts der Blutbehandlungsvorrichtung befindet, oder wobei die Blutbehandlungsvorrichtung ein Hämofilter zur Dialyse von Blut ist.

10. Blutbehandlungsvorrichtung nach Anspruch 7 oder 9, wobei die Blutbehandlungsvorrichtung eine Säule ist und mit Vollblut perfundiert ist.

11. Extrakorporaler Blutkreislauf, umfassend eine Blutbehandlungsvorrichtung nach einem der Ansprüche 1 bis 10, wobei der extrakorporale Blutkreislauf Mittel zum Transportieren von Blut oder Blutplasma von dem Gefäßsystem des Patienten zu der Blutbehandlungsvorrichtung mit einer definierten Flussrate und Mittel zum Rückführen des behandelten Blutes oder Blutplasmas zurück zu dem Patienten umfasst.

12. Blutbehandlungsvorrichtung nach einem der vorhergehenden Ansprüche zur Verwendung als ein Medikament bei der Behandlung und/oder Vorbeugung von:
a. einem medizinischen Zustand, der mit erhöhten asymmetrischen Dimethylarginin(ADMA) - und/oder Monomethylarginin(MMA)-Spiegeln im Blut oder Blutplasma eines Subjekts im Vergleich zu gesunden Kontrollen verbunden ist, und/oder
b. einem medizinischen Zustand, der mit einer verminderten Produktion und/oder Bioverfügbarkeit von Stickstoffoxid (NO) im Vergleich zu gesunden Kontrollen verbunden ist.

13. Blutbehandlungsvorrichtung zur Verwendung als Medikament nach Anspruch 12, wobei der medizinische Zustand Folgendes ist:
a. eine Gefäß- und/oder Herzerkrankung, bevorzugt ausgewählt aus der Gruppe, bestehend aus koronarer arterieller Erkrankung, peripherer atrialer Erkrankung, cerebraler arterieller Erkrankung, Herzinsuffizienz, Dyslipidämien, arterieller Hypertonie, pulmonaler Hypertonie, Eklampsie, digitalen Ulzera, erektiler Dysfunktion, Migräne, traumatischen Gefäßverletzungen, Transplantatvaskulopathien, mikrovaskulären Verletzungen, myokardialem Remodeling und vaskulärem Remodeling, Ischämie-Reperfusionsverletzungen und/oder vasospastischen Erkrankungen.
b. vaskuläre und kardiale Komplikationen von Stoffwechselerkrankungen, wie Diabetes mellitus, Dyslipidämien, Hyperhomocysteinämie und/oder Hyperurikämie
c. eine Leber- und/oder Nierenerkrankung

14. Enzym, das asymmetrisches Dimethylarginin (ADMA) und/oder Monomethylarginin (MMA), ausgewählt aus Dimethylarginin-Dimethylaminohydrolase (DDAH) und/oder Alanin-Glyoxylat-Aminotransferase 2 (AGXT2), verstoffwechselt, zur Verwendung als Medikament bei der Behandlung und/oder Vorbeugung eines medizinischen Zustands, der mit erhöhten asymmetrischen Dimethylarginin(ADMA) - und/oder Monomethylarginin(MMA)-Spiegeln im Blut oder Blutplasma eines Subjekts im Vergleich zu gesunden Kontrollen verbunden ist, wobei das Enzym an einer Matrix einer Blutbehandlungsvorrichtung immobilisiert ist.

## Revendications

1. Dispositif de traitement du sang configuré pour réduire la quantité de diméthylarginine asymétrique (ADMA) et/ou de monométhylarginine (MMA) dans le sang ou le plasma sanguin d'une personne en ayant besoin dans un circuit sanguin extracorporel, dans lequel le dispositif comprend une matrice, et dans lequel ladite matrice comprend de la diméthylarginine diméthylaminohydrolase (DDAH) et/ou de l'alanine-glyoxylate aminotransférase 2 (AGXT2).

2. Dispositif de traitement du sang selon la revendication 1, dans lequel :
a. la diméthylarginine diméthylaminohydrolase (DDAH) est immobilisée sur la matrice et configurée pour hydrolyser la diméthylarginine asymétrique (ADMA) et/ou la monométhylarginine (MMA) par catabolisme de l'ADMA et/ou de la MMA en L-citrulline dans le sang ou le plasma sanguin, et/ou
b. l'alanine-glyoxylate aminotransférase 2 (AGXT2) est immobilisée sur la matrice et configurée pour métaboliser la diméthylarginine asymétrique (ADMA) et/ou la monométhylarginine (MMA) par transamination en acide alpha-céto-delta-(NN-diméthylguanidino)valérique (ADGV) et en acide alpha-céto-delta-(N-monométhylguanidino)valérique (MGV), respectivement, dans le sang ou le plasma sanguin.

3. Dispositif de traitement du sang selon l'une quelconque des revendications précédentes, dans lequel la diméthylarginine diméthylaminohydrolase (DDAH) comprend ou est constituée d'isoforme 1 (DDAH1) et/ou d'isoforme 2 (DDAH2).

4. Dispositif de traitement du sang selon l'une quelconque des revendications précédentes, dans lequel la matrice comprend un support auquel sont liées la diméthylarginine diméthylaminohydrolase (DDAH) et/ou l'alanine-glyoxylate aminotransférase 2 (AGXT2), dans lequel le support comprend ou est constitué d'un matériau choisi dans le groupe constitué par une résine, une bille, un mat de fibres, un matériau non tissé, une mousse poreuse et une membrane, telle qu'une membrane tubulaire, à fibres creuses ou à feuille plate.

5. Dispositif de traitement du sang selon la revendication précédente, dans lequel le support et/ou la matrice comprennent ou sont constitués d'au moins un polymère, de préférence choisi dans le groupe constitué par l'alginate, le chitosane, la chitine, le collagène, la carraghénine, la gélatine, l'amidon, la pectine, la cellulose, l'agarose, le dextrose et le sépharose ; un matériau inorganique, de préférence choisi dans le groupe constitué par la céramique, la célite, la silice, le verre, le charbon actif et le charbon de bois ; ou un polymère synthétique.

6. Dispositif de traitement du sang selon l'une quelconque des revendications précédentes, dans lequel la diméthylarginine diméthylaminohydrolase (DDAH) et/ou l'alanine-glyoxylate aminotransférase 2 (AGXT2) sont liées de manière covalente à la matrice, par exemple par réticulation, ou sont liées de manière non covalente à la matrice, par exemple par des interactions ioniques ou une étiquette d'affinité.

7. Dispositif de traitement du sang selon l'une quelconque des revendications précédentes, dans lequel le dispositif de traitement du sang est situé dans un circuit sanguin extracorporel à travers lequel passe le sang du patient, dans lequel ledit circuit comprend un moyen pour transporter le sang du système vasculaire du patient vers le dispositif de traitement du sang à un débit défini et pour renvoyer le sang traité au patient.

8. Dispositif de traitement du sang selon la revendication précédente, dans lequel le circuit sanguin extracorporel dans lequel est situé le dispositif de traitement du sang comprend également un séparateur de plasma, tel qu'un filtre à plasma ou un système de séparation de plasma à base de centrifugeuse ou une combinaison de ceux-ci (par exemple un séparateur à disque), qui permet la séparation du plasma du sang, et dans lequel le dispositif de traitement du sang est situé en aval d'une sortie de plasma du séparateur de plasma.

9. Dispositif de traitement du sang selon la revendication 7, dans lequel le circuit sanguin extracorporel dans lequel est situé le dispositif de traitement du sang comprend également un hémofiltre pour la dialyse du sang qui est situé en amont ou en aval du dispositif de traitement du sang, ou dans lequel le dispositif de traitement du sang est un hémofiltre pour la dialyse du sang.

10. Dispositif de traitement du sang selon les revendications 7 ou 9, dans lequel le dispositif de traitement du sang est une colonne et est perfusé avec du sang complet.

11. Circuit sanguin extracorporel comprenant un dispositif de traitement du sang selon l'une quelconque des revendications 1 à 10, dans lequel le circuit sanguin extracorporel comprend un moyen pour transporter le sang ou le plasma sanguin du système vasculaire du patient vers le dispositif de traitement du sang à un débit défini et un moyen pour renvoyer le sang ou le plasma sanguin traité au patient.

12. Dispositif de traitement du sang selon l'une quelconque des revendications précédentes, pour une utilisation comme médicament dans le traitement et/ou la prévention de :
a. une pathologie médicale associée à des taux élevés de diméthylarginine asymétrique (ADMA) et/ou de monométhylarginine (MMA) dans le sang ou le plasma sanguin d'un sujet, par rapport à des témoins sains, et/ou
b. une pathologie médicale associée à une production et/ou une biodisponibilité d'oxyde nitrique (NO) altérées par rapport à des témoins sains.

13. Dispositif de traitement du sang pour une utilisation comme médicament selon la revendication 12, dans lequel la pathologie médicale est :
a. une maladie vasculaire et/ou cardiaque, de préférence choisie dans le groupe constitué par une maladie coronarienne, une maladie auriculaire périphérique, une maladie artérielle cérébrale, une insuffisance cardiaque, des dyslipidémies, une hypertension artérielle, une hypertension pulmonaire, une éclampsie, des ulcères digitaux, une dysfonction érectile, des migraines, des lésions vasculaires traumatiques, des vasculopathies de transplantation, des lésions microvasculaires, un remodelage myocardique et un remodelage vasculaire, des lésions d'ischémie-reperfusion et/ou des maladies vasospastiques.
b. complications vasculaires et cardiaques de maladies métaboliques, telles que le diabète sucré, les dyslipidémies, l'hyperhomocystéinémie et/ou l'hyperuricémie
c. une maladie hépatique et/ou rénale

14. Enzyme qui métabolise la diméthylarginine asymétrique (ADMA) et/ou la monométhylarginine (MMA), choisie parmi la diméthylarginine diméthylaminohydrolase (DDAH) et/ou l'alanine-glyoxylate aminotransférase 2 (AGXT2), pour une utilisation comme médicament dans le traitement et/ou la prévention d'une pathologie médicale associée à des taux élevés de diméthylarginine asymétrique (ADMA) et/ou de monométhylarginine (MMA) dans le sang ou le plasma sanguin d'un sujet, par rapport à des témoins sains, dans lequel ladite enzyme est immobilisée sur une matrice d'un dispositif de traitement du sang.
